(19) Europäisches Patentamt
European Patent Office
Office européen des brevets

(11) Numéro de publication: **0 124 407**
**B1**

(12) # FASCICULE DE BREVET EUROPÉEN

(45) Date de publication du fascicule du brevet: **23.08.89**

(51) Int. Cl.⁴: **C 07 D 333/24**, C 07 C 51/347, C 07 D 317/60

(21) Numéro de dépôt: **84400689.0**

(22) Date de dépôt: **06.04.84**

(54) **Procédé de préparation d'acides alkanoiques.**

(30) Priorité: **29.04.83 FR 8307137**

(43) Date de publication de la demande:
**07.11.84 Bulletin 84/45**

(45) Mention de la délivrance du brevet:
**23.08.89 Bulletin 89/34**

(84) Etats contractants désignés:
**BE CH DE FR GB IT LI NL**

(56) Documents cité:
**EP-A-0 032 374**
**FR-A-2 415 109**
**FR-A-2 470 127**
**GB-A-2 067 988**

**Le dossier contient des informations techniques présentées postérieurement au dépôt de la demande et ne figurant pas dans le présent fascicule.**

(73) Titulaire: **SOCIETE FRANCAISE HOECHST Société anonyme dite:, 3, avenue du Général de Gaulle, F-92800 Puteaux (FR)**

(72) Inventeur: **Christidis, Yani, 53, Boulevard de la Villette, F-75019 Paris (FR)**
Inventeur: **Vallejos, Jean- Claude, 9, rue Labat, F-75018 Paris (FR)**

(74) Mandataire: **Rinuy, Santarelli, 14, avenue de la Grande Armée, F-75017 Paris (FR)**

EP 0 124 407 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

## Description

La présente invention concerne un procédé de fabrication industrielle d'acides alkanoïques, plus particulièrement elle se rapporte à un procédé de fabrication d'acides acétiques substitués, ci-après désignés acides arylacétiques, de formule générale I,

$$\underset{\displaystyle R}{\text{Ar-}\overset{}{\underset{|}{C}}\text{H-COO-H}} \qquad I$$

dans laquelle R représente un atome d'hydrogène ou un radical alkyle en $C_1$-$C_4$ et Ar représente le radical: thiényle-2.

Les acides arylacétiques de formule générale I sont largement décrits dans la littérature. Ce sont notamment des matières premières intéressantes pour la synthèse de substances pharmaceutiques actives.

Il est donc d'un grand intérêt de pouvoir les préparer d'une manière aussi rationnelle que possible.

On connaît de très nombreuses méthodes pour préparer des acides acétiques substitués. Parmi celles-là, la réaction de Willgerodt-Kindler qui autorise la transformation d'une arylalkylcétone en acide arylalkylcarboxylique est l'une des plus connues. Toutefois, elle ne fournit généralement que dés rendements modérés à médiocres et elle conduit à des produits secondaires soufrés responsables d'une pollution inacceptable aujourd'hui. Pour remédier à ces inconvénients, on s'est adressé à des méthodes plus élaborées nécessitant l'emploi de plusieurs stades réactionnels telles que notamment l'hydrogénolyse, soit chimique, soit catalytique, d'acides arylglycoliques, d'acides o-acylé arylglycoliques ou d'acides aryl-2 halogéno-2 alkanoïques, issus dans certains cas de la condensation de l'acide glyoxylique sur le dérivé aromatique correspondant (demandes de brevet français n° 2 415 109, 2 470 127, demandes de brevet japanais n° 73-08775, 74-49954). Ces méthodes, dont certaines sont très récentes, exigent comme matière première de départ des produits rarement disponibles sur le marché ce qui nécessite leur préparation préalable avec les inconvénients que cela comporte.

Or, la Demanderesse a découvert avec étonnement qu'il était possible d'obtenir des acides arylacétiques de formule générale I, en un seul stade, par réaction à chaud, en milieu acide, en présence de phosphore rouge et de quantités catalytiques d'iode ou d'acide iodhydrique, d'un acide carboxylique alpha carbonylé de formule générale II, R-CO-COOH, dans laquelle R a la signification donnée précédemment avec du thiophène.

Malgré que corrélativement à la formation d'une mole d'acide-arylacétique de formule générale I, il se forme une mole d'eau, il est parfois avantageux de mettre en oeuvre le procédé de la présente invention en présence de faibles quantités d'eau supplémentaires.

Plus particulièrement, le procédé selon la présente invention consiste à faire réagir, à une température comprise entre 30 et 100°C, en milieu acide, une mole d'un acide carboxylique alpha carbonylé, de formule générale II, avec de une à 10 moles de thiophène, en présence de 0,5 à 5 atome-grammes de phosphore rouge, de 0,5 à 2 moles d'eau et de 0,01 à 0,1 mole d'iode ou d'acide iodhydrique, éventuellement au sein d'un solvant organique compatible tel que l'acide acétique, puis à isoler l'acide cherché de formule générale I par des moyens connus en soi.

Avantageusement, le procédé selon la présente invention consiste à faire réagir, à une température comprise entre 50 et 80°C, en milieu acide, une mole d'acide glyoxylique ou d'acide pyruvique, avec de une à 10 moles de thiophène, en présence de 0,5 à 3 atome-grammes de phosphore rouge, de 0,5 à 2 moles d'eau et de 0,025 à 0,05 mole d'iode ou d'acide iodhydrique éventuellement au sein d'acide acétique, puis à isoler l'acide cherché de formule I, par des moyens connus en soi.

L'acide iodhydrique est l'acide iodhydrique commercial en solution aqueuse à 57 % en poids.

Selon une variante, on peut mettre en oeuvre le procédé de la présente invention en présence de 0,001 à 1 mole, avantageusement de 0,005 à 0,3 mole d'un acide organique sulfonique tel que l'acide méthanesulfonique par mole d'acide carboxylique, alpha carbonylé de formule générale II, engagé. La durée réactionnelle du procédé de la présente invention est fonction des substrats mis en oeuvre; habituellement cette durée est comprise entre 4 et 20 heures.

Préférentiellement, l'acide carboxylique alpha carbonylé de formule générale II est l'acide glyoxylique ou l'acide pyruvique. L'acide glyoxylique utilisé est généralement de l'acide glyoxylique en solution aqueuse à 80 % en poids.

En fin de réaction, l'acide arylacétique cherché de formule générale I est isolé du milieu réactionnel par des moyens connus en soi. Généralement, on élimine du milieu réactionnel ramené à la température ambiante le phosphore non transformé par filtration, puis on traite le filtrat avec de l'acétate de sodium pour neutraliser l'acide iodhydrique et/ou l'acide organique sulfonique éventuellement présents.

Le filtrat est ensuite concentré sous vide à température contrôlée pour n'éliminer que le solvant réactionnel et/ou le produit de départ qui n'a pas réagi. Le concentré ainsi obtenu est dissous dans un solvant organique compatible, non-miscible à l'eau et la solution obtenue est lavée d'abord avec de l'eau contenant en solution du métabisulfite de sodium puis à l'eau, enfin elle est concentrée à sec sous vide. On isole ainsi l'acide arylacétique cherché de formule générale I qui pourra être si nécessaire purifié par recristallisation.

Avec certains substrats, il est parfois avantageux d'isoler l'acide arylacétique cherché sous forme de sel sodique, puis de déplacer ce sel par de l'acide chlorhydrique concentré.

Le procédé de la présente invention permet d'obtenir, entre autres, les acides suivants:

- acide thiophène-2 acétique;
- acide thiophène méthyl-2 acétique.

Tous ces acides sont des matières premières précieuses pour la préparation de substances à activités thérapeutiques. Un procédé analogue pour la synthèse d'acide arylacétiques est décrit dans la demande divisionnaire EP-A-2 657 930.

Les exemples suivants sont donnés à titre explicatif et nullement limitatif de l'invention.

**Exemple 1:**

On chauffe pendant 6 heures à 50°C sous agitation une suspension de:

- 46,3 g (0,5 mole) d'acide glyoxylique à 80 % en poids dans l'eau;
- 210 g (2,5 moles) de thiophène;
- 1,65 $cm^3$ (0,0125 mole) d'acide iodhydrique à 57 %, d = 1,7;
- 0,25 g (0,0025 mole) d'acide méthanesulfonique à 98 %;
- 23,3 g (0,75 at.g.) de phosphore rouge;
- 250 $cm^3$ d'acide acétique pur cristallisable.

Puis le milieu réactionnel refroidi à la température ambiante est filtré pour éliminer le phosphore qui n'a pas réagi. Le filtrat traité par 1,23 g (0,015 mole) d'acétate de sodium pur sec est ensuite concentré sous un vide de 20 mm Hg jusqu'à atteindre une température de 60°C. Après refroidissement à la température ambiante, on introduit dans ce milieu concentré, 125 $cm^3$ de dichloroéthane, on décante, puis on lave la phase organique avec une solution de 80 g d'eau contenant 15 g de chlorure de sodium et 3 g de métabisulfite de sodium. La phase organique est ensuite concentrée à sec sous vide et le résidu huileux (36,5 g) est distillé sous vide (0,6 mm Hg) (0,8 mB). On recueille ainsi 35,5 g (0,25 mole) d'acide thiophène-2 acétique distillant à 94°C et présentant un point de fusion de 62 ± 1°C. Le rendement s'établit à 50 % de la théorie calculée par rapport à l'acide glyoxylique mis en oeuvre.

**Exemple 2:**

On chauffe pendant 10 heures à 75°C sous agitation une suspension de:

- 44 g (0,5 mole) d'acide pyruvique;
- 420 g (5 moles) de thiophène;
- 3,3 $cm^3$ (0,025 mole) d'acide iodhydrique à 57 %, d = 1,7;
- 13 g (0,13 mole) d'acide méthanesulfonique à 98 %;
- 46,5 g (1,5 at.g.) de phosphore rouge;
- 200 $cm^3$ d'acide acétique pur cristallisable;
- 10 g d'eau.

Puis le milieu réactionnel refroidi à la température ambiante est filtré. Le filtrat traité par 13,2 g (0,161 mole) d'acétate de sodium pur sec est ensuite concentré à sec sous vide.

Le résidu huileux est repris à l'éther sulfurique, puis cette solution est lavée avec de l'eau contenant en solution 3 % en poids de métabisulfite de sodium, et elle est ensuite concentrée à sec sous vide. On obtient ainsi 65 g d'huile que l'on distille sous vide. On recueille 48,9 g d'acide méthyl-2 (thiényl-2)-2 acétique distillant sous un vide de 8 mm Hg (10,64 mB) à 144 ± 2°C. Le rendement s'établit à 62,6 % de la théorie calculée par rapport à l'acide pyruvique mis en oeuvre.

Il va de soi que la présente invention n'a été décrite qu'à titre purement explicatif et nullement limitatif et que modification utile pourra y être apportée sans sortir de son cadre.

**Revendications**

1. Procédé de préparation d'acides acétiques substitués de formule générale (I) Ar-CHR-COOH, dans laquelle R représente un atome d'hydrogène ou un radical alkyle en $C_1$-$C_4$ et Ar représente le radical thiényle-2, par réaction à chaud, en milieu acide, d'un acide alpha carbonylé de formule générale (II) R-CO-COOH dans laquelle R a la signification ci-dessus avec du thiophène, du phosphore rouge, et des quantités catalytiques d'iode ou d'acide iodhydrique et en présence ou non d'un acide organique sulfonique, caractérisé par le fait que la réaction est réalisée en un seul stade.

2. Procédé selon la revendication 1, caractérisé par le fait que l'on fait réagir à une température comprise entre 30 et 100°C, en milieu acide, une mole d'acide carboxylique alpha carbonylé de formule générale III telle que définie à la revendication 1, avec de 1 à 10 moles d'un dérivé insaturé à caractère aromatique tel que défini à la revendication 1, en présence de 0,5 à 5 atomegrammes de phosphore rouge, de 0,5 à 2 moles d'eau et de 0,01 à 0,1 mole d'iode ou d'acide iodhydrique, éventuellement au sein d'acide acétique.

3. Procédé selon l'une quelconque des revendications 1 ou 2, caractérisé par le fait que l'on fait réagir à une température comprise entre 50 et 80°C, en milieu acide, une mole d'acide glyoxylique ou d'acide pyruvique avec de 1 à 10 moles d'un dérivé insaturé à caractère aromatique tel que défini à la revendication 1, en présence de 0,5 à 3 atome-grammes de phosphore rouge, de 0,5 à 2 moles d'eau et de 0,025 à 0,05 mole d'iode ou d'acide iodhydrique, éventuellement au sein d'acide acétique.

4. Procédé selon l'une quelconque des revendications 1 à 3, caractérisé par le fait qu'il est

réalisé en présence de 0,001 à 1 mole d'un acide organique sulfonique par mole d'acide carboxylique alpha carbonylé mis en oeuvre.

5. Procédé selon la revendication 4, caractérisé par le fait que l'acide organique sulfonique utilisé est l'acide méthanesulfonique.

6. Application du procédé selon l'une quelconque des revendications 1 à 5 à la fabrication de l'acide thiophène-2 acétique, caractérisée par le fait que le dérivé insaturé à caractère aromatique mis en oeuvre est le thiophène et l'acide carboxylique alpha carbonylé utilisé est l'acide glyoxylique.

7. Application du procédé selon l'une quelconque des revendications 1 à 5 à la fabrication de l'acide thiophène-2 méthyl-2 acétique, caractérisée par le fait que le dérivé insaturé à caractère aromatique mis en oeuvre est le thiophène et que l'acide carboxylique alpha carbonylé utilisé est l'acide pyruvique.

**Patentansprüche**

1. Verfahren zur Herstellung von substituierten Essigsäuren der allgemeinen Formel (I) Ar-CHR-COOH,

in der R ein Wasserstoffatom oder einen $C_1$-$C_4$ Alkylrest und Ar den Rest Thienyl-2 bedeutet, wobei eine carbonylierte Alpha-Säure der allgemeinen Formel (II) R-CO-COOH,

in der R die vorstehend angegebene Bedeutung hat, bei Hitze, unter saueren Bedingungen und gegebenenfalls in Gegenwart einer organischen Sulfonsäure mit Thiophen, rotem Phosphor und katalytischen Mengen von Jod oder Jodwasserstoffsäure umgesetzt wird, dadurch gekennzeichnet, daß die Umsetzung in einem einzigen Reaktionsschritt erfolgt.

2. Verfahren nach Anspruch 1, dadurch gekennzeichnet, daß 1 Mol einer carbonylierten Alpha-Carbonsäure der allgemeinen Formel II, wie sie in Anspruch 1 angegeben ist, bei einer Temperatur zwischen 30° und 100°C unter saueren Bedingungen und in Gegenwart von 0,5 bis 5 Grammatom rotem Phosphor, 0,5 bis 2 Mol Wasser und 0,01 bis 0,1 Mol Jod oder Jodwasserstoffsäure sowie gegebenenfalls Essigsäure mit 1 bis 10 Mol eines ungesättigten aromatischen Derivats, wie dies in Anspruch 1 angegeben ist, umgesetzt wird.

3. Verfahren nach einem der Ansprüche 1 oder 2, dadurch gekennzeichnet, daß 1 Mol Glyoxalsäure oder Brenztraubensäure bei einer Temperatur zwischen 50° und 80°C unter saueren Bedingungen und in Gegenwart von 0,5 bis 3 Grammatom rotem Phosphor, 0,5 bis 2 Mol Wasser und 0,025 bis 0,05 Mol Jod oder Jodwasserstoffsäure sowie gegebenenfalls Essigsäure mit 1 bis 10 Mol eines ungesättigten aromatischen Derivats, wie dies in Anspruch 1 angegeben ist, umgesetzt wird.

4. Verfahren nach einem der Ansprüche 1 bis 3, dadurch gekennzeichnet, daß pro Mol der eingesetzten carbonylierten Alpha-Carbonsäure 0,001 bis 1 Mol einer organischen Sulfonsäure eingesetzt wird.

5. Verfahren nach Anspruch 4, dadurch gekennzeichnet, daß die eingesetzte organische Sulfonsäure Methansulfonsäure ist.

6. Anwendung des Verfahrens nach einem der Ansprüche 1 bis 5 zur Herstellung von Thiophen-2-essigsäure, dadurch gekennzeichnet, daß das eingesetzte ungesättigte aromatische Derivat Thiophen und die verwendete carbonylierte Alpha-Carbonsäure Glyoxalsäure ist.

7. Anwendung des Verfahrens nach einem der Ansprüche 1 bis 5 zur Herstellung von Thiophen-2-methyl-2-essigsäure, dadurch gekennzeichnet, daß das eingesetzte ungesättigte aromatische Derivat Thiophen und die verwendete carbonylierte Alpha-Carbonsäure Brenztraubensäure ist.

**Claims**

1. A process for the preparation of substituted acetic acids having the general formula (I) Ar-CHR-COOH in which R represents a hydrogen atom or a $C_1$-$C_4$-alkyl group, and Ar represents a 2-thienyl group, by reacting under heat, in an acid medium, an alpha carbonylated carboxylic acid having the general formula (II) R-CO-COOH in which R is as defined above with thiophene, the reaction being performed in the presence of red phosphous and of catalytic quantities of iodine or hydroiodic acid, and optionally in the presence of an organic sulfonic acid, characterised in that the reaction is performed in a single step.

2. A process according to claim 1, characterised in that one mole of alpha carbonylated carboxylic acid having the general formula II as defined in claim 1 is reacted at a temperature of between 30° and 100°C in an acid medium with from 1 to 10 moles of an unsaturated derivative of an aromatic nature as defined in claim 1, in the presence of 0.5 to 5 gram-atoms of red phosphorus, 0.5 to 2 moles of water and from 0.01 to 0.1 mole of iodine or hydriodic acid, the process optionally being performed in acetic acid.

3. A process according to claim 1 or claim 2, characterised in that 1 mole of glyoxylic acid or pyruvic acid is reacted at a temperature of between 50° and 80°C in an acid medium with from 1 to 10 moles of an unsaturated derivative of an aromatic nature such as defined in claim 1, in the presence of 0.5 to 3 atom-grams of red phosphorus, from 0.5 to 2 moles of water and from 0.025 to 0.05 mole of iodine or hydriodic acid, the process optionally being performed in acetic acid.

4. A process according to any one of claims 1 to 3, characterised in that the process is carried out in the presence of 0.001 to 1 mole of an organic sulfonic acid per mole of alpha carboxylated carboxylic acid used.

5. A process according to claim 4, characterised in that the organic sulfonic acid used is methanesulfonic acid.

6. Use of a process as claimed in any one of claims 1 to 5 for the preparation of 2-thiophene acetic acid, characterised in that the unsaturated derivative of aromatic nature is thiophene, and the alpha carbonylated carboxylic acid is glyoxylic acid.

7. Use of a process as claimed in any one of claims 1 to 5 for the preparation of 2-thiophene-2-methyl acetic acid, characterised in that the unsaturated derivative of aromatic nature is thiophene, and the alpha carbonylated carboxylic acid is pyruvic acid.